# EUROPEAN PATENT APPLICATION

(11) **EP 4 538 367 A2**
(43) Date of publication of application: **16.04.2025**
(21) Application number: 24201792.9
(22) Date of filing: 20.09.2024
(51) Int. Cl.: C12N 9/02, C12N 9/10, C12N 9/24, C12P 17/12, C12P 17/18

(54) **POLYPEPTIDES FOR USE IN THE SYNTHESIS OF KRATOM ALKALOIDS**

(30) Priority: 20.09.2023 US 202363583976 P
(71) Applicant: Mitradyne Corporation, Toronto, ON M6C 1R5 (CA)
(72) Inventor: CASARETTO, Jose, Guelph, N1G 5K3 (CA); AKTAR, Tariq A., Guelph, N1E 1B7 (CA); ROTHSTEIN, Steven, Guelph, N1G 1K4 (CA); SOUBEYRAND, Eric, Kitchener, N2P 1N9 (CA)
(74) Representative: Dehns

(57) **Abstract**

Described herein are polypeptides encoding enzymes for the synthesis of monoterpene indole alkaloids. For example, the polypeptide comprises or consists of the sequence of SEQ ID NO: 1-65, or a variant thereof having at least 80% sequence identity to SEQ ID NO: 1-65, or a fragment of the polypeptide, or the variant thereof.

## Description

### CROSS REFERENCE

The present application claims priority under the Paris Convention to US Patent Application Number 63/583,976, filed on September 20, 2023. The content of such prior application is incorporated herein by reference as if set forth in its entirety.

### TECHNICAL FIELD

The present invention relates to polypeptides. More specifically, the present invention is, in embodiments, concerned with the use of polypeptides encoding enzymes that can be used to synthesize indole alkaloid molecules and related products, methods, and uses.

### BACKGROUND

Opioids are one of the most commonly used analgesic agents in clinical practice. These compounds bind specific receptors in the central and peripheral nervous system to elicit analgesia.

Naturally occurring alkaloids (plant-derived amines) isolated from opium poppy such as morphine, codeine, and papaverine are the most widely known plant-derived analgesics. In addition, chemical manipulations of these opiate alkaloids have produced a range of semi-synthetic opioids also useful in clinical medicine such as diamorphine, dihydrocodeine, buprenorphine, naloxone, and oxycodone. During the past decades, however, several synthetic opioids were also produced either by chance or by design. Examples of these synthetic compounds are butorphanol, levorphanol, tramadol, methadone, pentazocine, alfentanil, fentanyl, sufentanil, and remifentanil. Their prolonged use, misuse without medical supervision, or non-medical use, can lead to opioid dependence, and in overdose cases can lead to death. In recent years, about half a million deaths worldwide are attributable to drug use. In most countries, about 70% of these deaths are related to opioids, with more than 30% of those deaths caused by overdose (WHO, 2022; CDC, 2022: Gardner et al., 2022). There are, however, some treatments for opioid dependence that can decrease the risk of overdose. For example, naloxone, a competitive opioid receptor antagonist (binds to receptors but does not produce any functional response and prevents an agonist from binding to that receptor), can reverse the depression of the respiratory and central nervous systems caused by opioids and is used to prevent death from an opioid overdose if administered in time (CDC, 2022).

Individuals who suffer from chronic pain or want to reduce dependence on opioids and opiates are also focusing on other options. One of those is the plant known as kratom [*Mitragyna speciosa* (Korth.)], a tropical tree native to Southeast Asia (Coe et al., 2019; Palamar, 2021). In the last decade, its use has increased in popularity, especially in the U.S and Europe, although the medicinal use of kratom as a local anesthetic and substitute for opium in Malaysia and Thailand has been documented nearly 100 years ago (Field, 1921). Kratom has been traditionally consumed as a tea, or by chewing leaves, however, other formulations are currently available, including capsules and whole powder extracts. Kratom extracts have demonstrated opioid-receptor mediated analgesic effects when administered; whereas low doses have stimulatory effects, larger doses are associated with opioid-like sedative effects. This activity has been attributed to alkaloids to this plant exclusively, and in particular to mitragynine and 7-hydroxymitragynine (Prozialeck et al., 2012). Both compounds can bind to the human opioid receptors, and function as partial agonists of the µ-opioid receptor (µOR) and weak antagonists at k-opioid and δ-opioid receptors (kOR, δOR) (Kruegel et al., 2016).

Unlike natural and clinically used synthetic opioids, kratom alkaloids do not promote β-arrestin recruitment to the opioid receptor when they activate the Gαi/o protein (Kruegel et al., 2016; Váradi et al., 2016). Several studies suggest that reduced recruitment of β-arrestin at the mu opioid receptors (pORs) and delta opioid receptor (δOR) is associated with weakened side effects caused by opioids such as respiratory depression (Gutridge et al., 2020; Mafi et al, 2020; Wilson et al., 2021). This mechanistic effect correlates with studies in animal models which have indicated that mitragynine has a low abuse potential (Hemby et al., 2019; Wilson et al., 2021). Nonetheless, this alkaloid can be metabolized into 7-hydroxymitragynine which exhibits greater potency than mitragynine and morphine (Avery et al., 2019; Kruegel and Grundmann 2018; Matsumoto et al., 2004), and is thought to be responsible for certain drug dependencies (Yusoff et al., 2016; Yue et al., 2018; Hemby et al., 2019; Gutridge et al., 2020). As a result, kratom has been subjected to the FDA's medical and safety evaluation and has prompted efforts to label 7-hydroxymitragynine and mitragynine Schedule I drugs by the Drug Enforcement Agency (Griffin and Webb, 2018). While not being illegal in the USA or most European countries, the possession and use of *Mitragyna speciosa* is controlled. In several countries of Europe, Southeast Asia, and Australia, kratom and/or mitragynine and 7-HMG are under control by narcotic laws due to their high misuse potential (Meireles et al., 2019).

Although mitragynine and 7-hydroxymitragynine have been the focus of multiple pharmacological investigations, kratom leaves are also able to produce 40 other alkaloids. The relative concentrations of such molecules depend on several factors including the variety of *Mitragyna speciosa,* (different varieties are found in different geographical region where the plant is grown), the harvest season, and the age of the leaves or plant (Adkins et al., 2011; Leon et al., 2009).

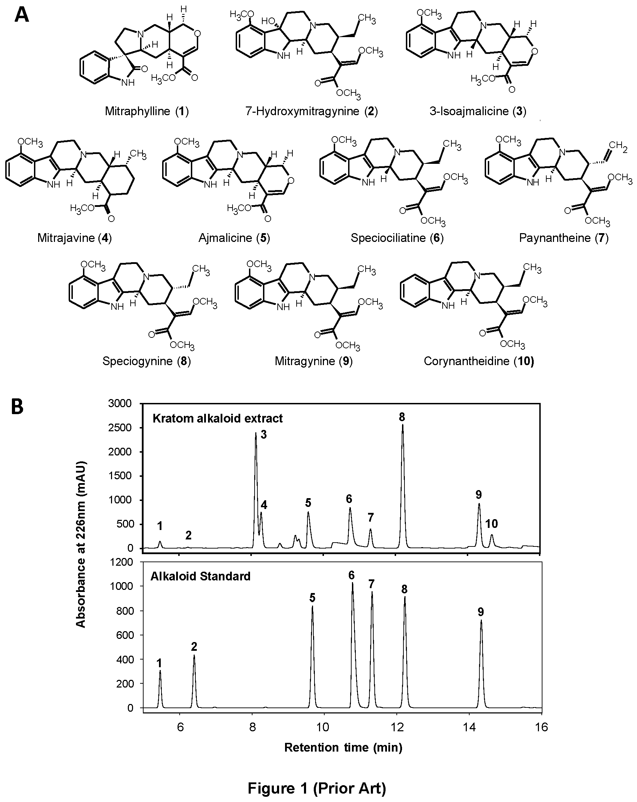

Kratom alkaloids are of the indole class of the corynanthe-type monoterpenoid alkaloids and oxindole alkaloids (Flores-Bocanegra et al., 2020). Besides the two already mentioned, other examples of such components are: paynantheine, speciociliatine, speciogynine, corynantheidine, corynoxine, corynoxine B, isocorynantheidine, mitraphilline (Sharma et al., 2019; **Figure 1****).** In addition, other compounds, such as flavonoids, terpenoids, and polyphenols have also been identified (Feng et al., 2017; Veeramohan et al., 2018).

The limited pharmacological data currently available for most of the kratom alkaloids warrants the investigation of pure alkaloids present in the plant and their significance in different products used medicinally. Several considerations indicate the need for more research on these molecules. One fact is that there exist differences in the genetics of kratom plants (e.g. different varieties) that influence the phytochemistry of the plant, hence, the same alkaloids are not present in the same proportion. Kratom products are sold under a variety of names, however, there is no information regarding the chemical composition of these preparations. Therefore there is a need for standardized formulations, especially if they are for medicinal use. For example, the relative potency of some kratom alkaloids on the opioid receptors is still unknown, therefore is difficult to predict the efficacy, reduced side effects, and potential health implications of products containing them.

The biosynthesis of the indole alkaloids relies on two precursors, tryptamine which is derived from the amino acid tryptophan providing the indol moiety to the structure, and secologanin, an iridoid monoterpene (Charoonratana et al., 2013). The biosynthesis pathway starts with the condensation reaction of tryptamine with secologanin to form strictosidine (Jumali et al., 2011), a precursor of hundreds of derivatives of monoterpene indole alkaloids in several plant families (Treimer & Zenk, 1979; de Luca et al., 2014).Geissoschizine and cathenamine are two examples of monoterpene alkaloids that can be synthesized starting from strictosidine. These alkaloids have been identified in fewer species, including kratom (de Luca et al., 2014; Sharma et al., 2019). Therefore, while the synthesis of indole alkaloids branches out from strictosidine to the diversification of these molecules among many plant species, those produced downstream in the biosynthetic pathway in M. *speciosa* are exclusive to this plant.

Recently, two enzymatic steps of intermediate molecules for the synthesis of mitragynine were described (Schotte et al., 2023), however all the enzyme-encoding genes associated with the synthesis pathway of indole alkaloids in kratom is still elusive.

Various kratom alkaloids and their use have been previously published as in the following examples.

U.S. Publication No. 8648090B2 describes indole alkaloid derivatives having an opioid receptor agonistic effect, their synthesis, and therapeutic compositions containing these derivatives, and methods of treating conditions with these compounds and therapeutic compositions, are provided.

U.S. Patent Publication US10751380B2 relates to pharmaceutical and nutraceutical compounds and methods for reducing inflammation, muscle spasms, and pain associated with cancer, trauma, medical procedure, neurological diseases, and disorders, as well as other conditions in subjects in need thereof. The disclosed invention further relates to *Mitragyna speciosa* and *Cannabis sativa* based compounds and synthetic analogs of the alkaloids found in said plants, where said compounds can also contain a pharmacologically inactive substance for modifying certain qualities of said compounds.

U.S. Patent Publications US10961244B2 and WO2017165738A1 relate to a compound having a structure related to mitragynine alkaloids or a pharmaceutically acceptable salt or ester, and a method of treating a subject afflicted with pain, a depressive disorder, a mood disorder or an anxiety disorder by administering the compound to the subject.

U.S. Patent Applications No. 2021/0113644A1 and WO2021076849A1 disclose compositions containing two purified kratom compounds chosen from the following: 7-Hydroxymitragynine, Mitragynine, Paynantheine, Speciociliatine, Speciogynine, Ajmalicine, Ciliaphylline, Corynantheidine, Corynoxine A, Corynoxine B, Isomitraphylline, Isorhynchophylline, Mitraphylline, Rhynchophylline, Speciophylline, Speciofoline, Epicatechin, 7-Hydroxyspecioliatine, 9-Hydroxycorynantheidine, Corynoxeine, Isopteropodine, Isorhynchophylline Oxindole, Tetrahydroalstonine, Mitragynine Oxindole B, and Mitragynine Oxindole A or the salts of these kratom compounds. The disclosure also relates to formulations, including pharmaceutical formulations, of such a composition and an excipient. Pharmaceutical formulation further containing a therapeutically effective amount of a serotonergic drug, a purified psilocybin derivative, a purified cannabinoid, or a purified terpene are disclosed. Also disclosed are methods of regulating the activity of a neurotransmitter receptor and methods of treating a psychological disorder, a compulsive disorder, or a depressive disorder.

U.S. Patent Publication No. 11046692B2 describes compounds formulae of mitragynine analogs and pharmaceutically acceptable derived salts that are useful for modulating opioid receptor activity. The provided compounds may have both agonistic and antagonistic effects on one or more opioid receptors. Methods of using the compounds for treating or managing pain are also described.

U.S. Patent Application No. 20220135564A1 describes a compound having the structure of a deuterated mitragynine or a pharmaceutically acceptable salt or ester thereof, and methods of using the compound to treat pain, depressive disorders, mood disorders, anxiety disorders, opioid use disorder, and opioid withdrawal symptoms.

U.S. Patent Application No. 20210179619A1 relates to mitragynine analogs for the treatment of pain, mood disorders and substance use disorders. The invention provides a compound having the structure of an indole alkaloid related to mitragynine, and a method of treating a subject afflicted with pain, a depressive disorder, a mood disorder or an anxiety disorder by administering the compound to the subject.

Patent Publication WO2022008955 describes preparations containing Mitragyne extract or its isolated alkaloids and cannabis extract or its isolated cannabinoids, and cosmetic and/or pharmaceutical preparation for topical use containing from about 0.00001 wt. -% to about 20 wt. -%, of one or more alkaloids of *Mitragyna speciosa* and from about 0.00001 wt. -% to about 20 wt. -% of one or more cannabinoids of *Cannabis indica* or *Cannabis sativa.* The preparation is intended for use in preventing and/or treating skin and mucosal disorders and conditions.

Patent Publication CN112094265 describes an extraction method of 7-OH-mitragynine, which comprises the following steps: (1) pulverizing dried kratom leaves, adding an extractant, carrying out ultrasonic extraction at normal temperature, and carrying out centrifugation and vacuum concentration to obtain a first concentrated solution; (2) adding an extraction agent into the first concentrated solution for extraction, and performing vacuum concentration to obtain a second concentrated solution; and (3) carrying out silica gel column chromatography separation on the second concentrated solution, and carrying out gradient elution with an eluent to obtain the 7-OH-mitragynine with high purity.

Research and potential utilization of individual or specific combinations of kratom alkaloids are impeded by the lack of understanding of their biosynthesis in the kratom plant. Therefore, there is a need to provide a useful alternative to overcome at least some of the deficiencies of the prior art.

### SUMMARY

In accordance with an aspect, there is provided a polypeptide encoding a strictosidine glucosidase that removes the glucose moiety from strictosidine to produce strictosidine aglycone.

In an aspect, the polypeptide is a strictosidine glucosidase from *Mitragyna speciosa.*

In an aspect, the polypeptide is comprising or consisting of a polypeptide having at least 80%, 85%, 90%, 91%, 92%, 93%, 94%, 95%, 96%, 97%, 98%, 99%, or 100% sequence identity to the sequence of any one or more of SEQ ID NO: 1-13, or a fragment of any thereof.

In an aspect, the polypeptide produces strictosidine-aglycone when reacted under suitable conditions in the presence of strictosidine.

In accordance with an aspect, there is provided a polypeptide encoding a geissoschizine synthase that opens the tetrahydropyran ring of strictosidine aglycone to produce geissoschizine.

In an aspect, the polypeptide is a geissoschizine synthase from *Mitragyna speciosa.*

In an aspect, the polypeptide is comprising or consisting of a polypeptide having at least 80%, 85%, 90%, 91%, 92%, 93%, 94%, 95%, 96%, 97%, 98%, 99%, or 100% sequence identity to the sequence of any one or more of SEQ ID NO: 14-22, or a fragment of any thereof.

In an aspect, the polypeptide produces geissoschizine when reacted under suitable conditions in the presence of strictosidine aglycone.

In accordance with an aspect, there is provided a polypeptide encoding a hydroxylase that hydroxylates the indole ring of coηnantheidine at the C9 position to produce 9-hydroxycorynantheidine.

In accordance with an aspect, the polypeptide encoding a hydroxylase hydroxylates the indole ring of mitragynine at the C7 position to produce 7-hydroxymitragynine.

In an aspect, the polypeptide is a hydroxylase from *Mitragyna speciosa.*

In an aspect, the polypeptide is comprising or consisting of a polypeptide having at least 80%, 85%, 90%, 91%, 92%, 93%, 94%, 95%, 96%, 97%, 98%, 99%, or 100% sequence identity to the sequence of any one or more of SEQ ID NO: 23-54, or a fragment of any thereof.

In an aspect, the polypeptide produces a 9-hydroxy-indole alkaloid, such as 9-hydroxycorynantheidine.

In an aspect, the polypeptide produces 7-hydroxymitragynine when reacted under suitable conditions in the presence of mitragynine.

In accordance with an aspect, there is provided a polypeptide encoding an O-methyltransferase that methylates the hydroxyl group at the C9 position of 9-hydroxycorynantheidine to produce mitragynine.

In an aspect, the polypeptide is an O-methyltransferase from *Mitragyna speciosa.*

In an aspect, the polypeptide is comprising or consisting of a polypeptide having at least 80%, 85%, 90%, 91%, 92%, 93%, 94%, 95%, 96%, 97%, 98%, 99%, or 100% sequence identity to the sequence of any one or more of SEQ ID NO: 55-65, or a fragment of any thereof.

In an aspect, the polypeptide produces mitragynine when reacted under suitable conditions in the presence of 9-hydroxycorynantheidine.

In an aspect, the polypeptide, variant, or fragment comprises up to about 100, about 150, about 200, about 250, about 300, about 350, about 360, about 370, about 380, about 390, about 400, about 410, about 420, about 430, about 440, about 450, or about 500 amino acids

In an aspect, the polypeptide is synthetic.

In an aspect, the polypeptide is recombinant.

In accordance with an aspect, there is provided a nucleic acid encoding the polypeptide described herein.

In an aspect, the nucleic acid is cDNA.

Polypeptides and nucleic acids of the invention may be isolated polypeptides and isolated nucleic acids.

In accordance with an aspect, there is provided a vector comprising the nucleic acid described herein.

In accordance with an aspect, there is provided a host cell comprising the vector described herein.

In accordance with an aspect, there is provided a host cell expressing the polypeptide described herein.

In an aspect, the host cell is a bacterial cell (e.g., *E. coli* or *Agrobacterium tumefaciens*), a yeast cell (e.g., *S. cerevisiae*), an algal cell, or a plant cell (e.g., *Nicotiana* spp.).

In an aspect, the host cell is provided in combination with an indole alkaloid precursor.

In an aspect, the indole alkaloid precursor is provided in the host cell culture medium.

In an aspect, the indole alkaloid precursor is expressed by the host cell.

In an aspect, the indole alkaloid precursor is strictosidine.

In accordance with an aspect, there is provided an expression system comprising the polypeptide; the nucleic acid, the vector; or the host cell described herein.

In accordance with an aspect, there is provided a system for modifying an indole alkaloid the system comprising the polypeptide described herein.

In an aspect, the system is cell-free.

In accordance with an aspect, there is provided a method for modifying an indole alkaloid, wherein the method comprises contacting the indole alkaloid with a polypeptide described herein.

In an aspect, the method is a recombinant method comprising expressing a polypeptide described herein in a cell in the presence of an indole alkaloid precursor.

In an aspect, the method comprises combined enzymatic reaction steps.

In accordance with an aspect, there is provided a method of producing strictosidine aglycone, 4,21-dehydrogeissoschizine, geissoschizine, corynantheidine, 9-hydroxycorynantheidine, mitragynine, 7-hydroxymitragynine, speciociliatine, speciogynine, and paynantheine, the method comprising carrying out combined enzymatic reactions with the polypeptides described herein.

In an aspect, the final indole alkaloid is substantially pure, for example, at least about 95%, about 96%, about 97%, about 98%, about 99%, about 99.5%, or about 99.9% pure.

In accordance with an aspect, there is provided a pharmaceutical composition comprising the indole alkaloid described herein and at least one pharmaceutically acceptable carrier.

In accordance with an aspect, there is provided a natural health product comprising the indole alkaloid described herein, such as a supplement, beverage, or food.

In accordance with an aspect, there is provided a use of the indole alkaloid described herein in a pharmaceutical or natural health product.

Other features and advantages of the present invention will become apparent from the following detailed description. It should be understood, however, that the detailed description and the specific examples while indicating embodiments of the invention are given by way of illustration only since various changes and modifications within the spirit and scope of the invention will become apparent to those skilled in the art from the detailed description.

### BRIEF DESCRIPTION OF THE DRAWINGS

The present invention will be further understood from the following description concerning the Figures:
**Figure 1****.** Depicts alkaloids detected in a crude kratom leaf extract (prior art). **(A)** Chemical structure of alkaloids detected in the extracts . **(B)** Representative HPLC chromatogram illustrating the alkaloid profile of a final crude extract and detected at 226 nm. The different peaks (top panel) were compared with available standards (bottom panel) and then collected and analyzed via mass spectrometry. The peaks represent, respectively,: 1, mitraphylline; 2, 7-hydroxymitragynine; 3, 3-isoajmalicine; 4, mitrajavine; 5, ajmalicine; 6, speciociliatine; 7, paynantheine; 8, speciogynine, 9, mitragynine and 10, coηnantheidine.

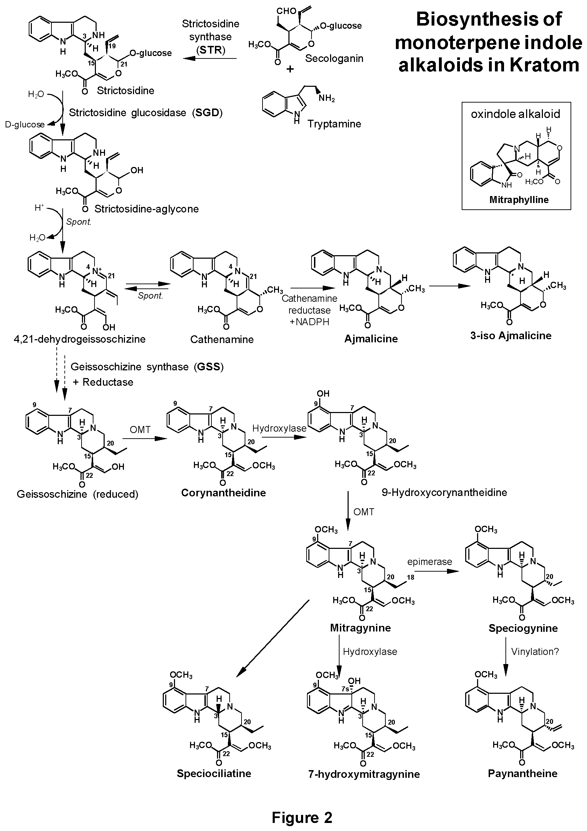

**Figure 2****.** Is a scheme depicting a biosynthetic pathway of monoterpene alkaloids in *Mitragyna speciosa.* The inset shows an example of oxindole alkaloids from kratom which are synthesized via a separate pathway.

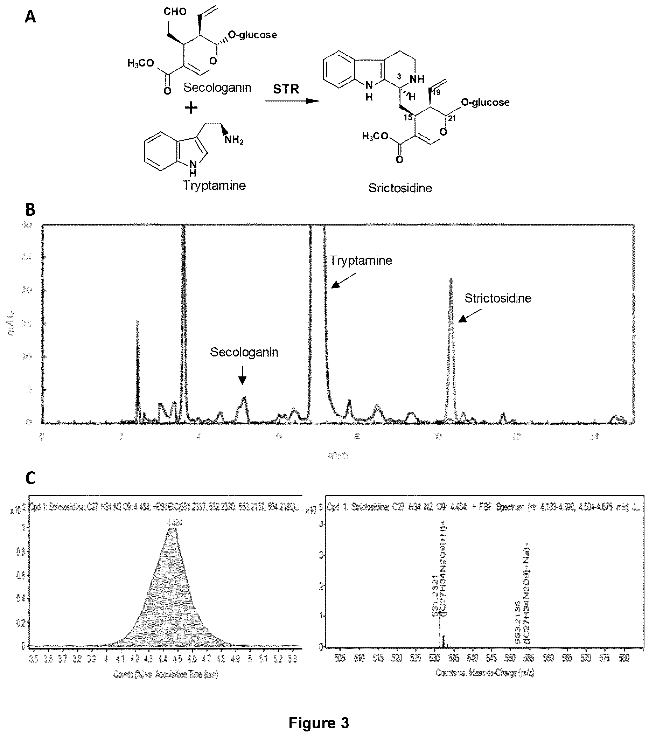

**Figure 3****. (A)** is a scheme depicting the synthesis of strictosidine by MsSTR-1 and the reaction catalyzed by the identified sequence. **(B)** is a graph showing overlapped HPLC chromatograms showing the reactions with recombinant enzyme (MsSTR-1/c133034 without boiling (light line profile showing strictosidine) or after boiling the enzyme (darker line profile with the peaks of the substrates secologanin and tryptamine). (C) LC includes mass spectra obtained with the standard and the peak collected from the HPLC, confirming the identity of the product as strictosidine.

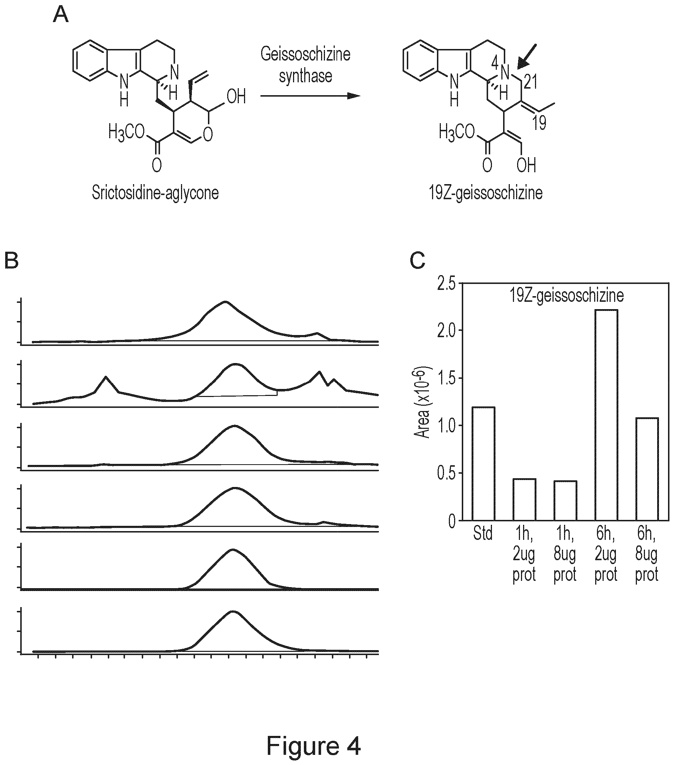

**Figure 4****.** (A) is a scheme depicting the conversion reaction of stictosidine aglycone to 19Z-geissoschizine catalyzed by the identified sequence. (B) includes graphs of HPLC chromatograms showing the products of the reactions obtained with different amounts of recombinant enzyme (MsGSS-1/c101879) and in different reaction times. (C) is a graph of the calculated area for the HPLC peaks for the products which co-elute with the standard.

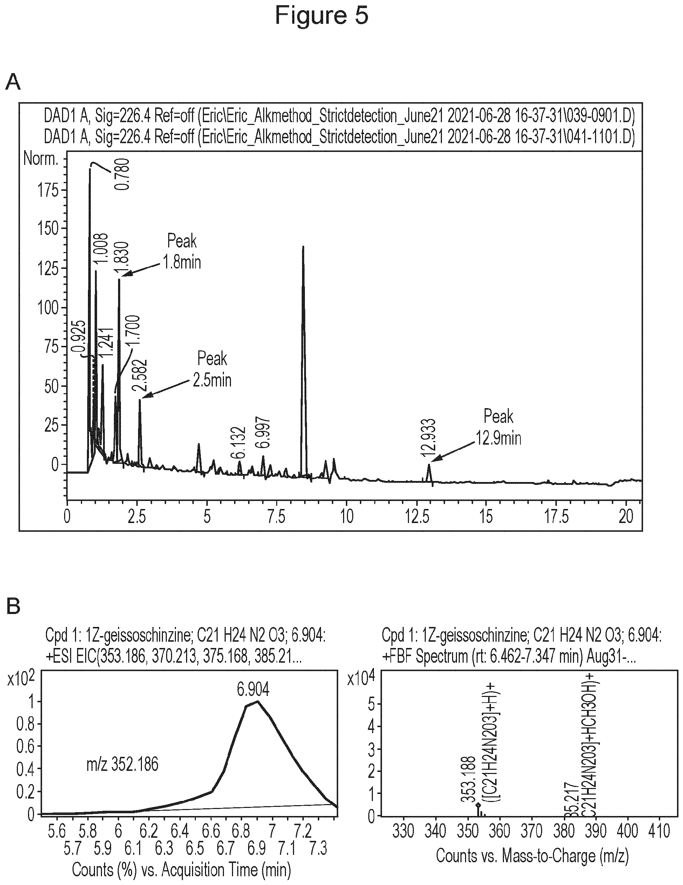

**Figure 5****.** (A) is a graph of the reaction assay quantified with the HPLC method as described in Figure 4 showing overlapped HPLC chromatograms showing the reactions with recombinant enzyme (MsGSS-1/c101879 without boiling (line profile with the peaks indicated by the arrows) or after boiling the enzyme as control (base line). The three peaks indicated by arrows were collected for identification by LC-MS. (B) is a graph of the mass spectra profile obtained with the peak eluted at 12.9 min from the HPLC, confirming the identity of the product as 19Z-geissoschizine.

### DETAILED DESCRIPTION

The diverse biological activities of monoterpene indole alkaloids have prompted the utilization of plants containing these as medicinal plants and as ingredients in products for treating pain, or addiction problems. Some show promise as important compounds for the development of new pharmacological agents or nutraceuticals for the treatment of different medical conditions. However, several obstacles have limited the potential applications of these compounds:
- Apart from a small number of alkaloids that are relatively abundant in kratom leaves, most of these monoterpene alkaloids often exist at trace levels or are not present in all kratom varieties.
- Extraction of these scarce but valuable alkaloids from the plant poses a significant challenge and their purification from mixtures is also complicated and costly.
- Isolation from the kratom plant implies access to biomass which may not available due to legal or operational concerns.
- In many cases, organic chemical synthesis is not an amenable cost-effective approach.

Described herein are enzymes that can serve as production catalysts for the *in vitro* and *in vivo* modification of indole alkaloid precursors such as strictosidine to produce unique indole alkaloids present in the plant *M*. *speciosa.* In addition, the polypeptide sequences described herein can be used in reactions for modifying similar molecules and generate novel synthetic indole alkaloid derivatives presenting similar, attenuated, or enhanced analgesic activities.

Thus, described herein are polypeptides that can act as enzymes, and related methods for the synthesis of monoterpene indole alkaloids. These polypeptides are typically derived from plant sources, such as *Mitragyna speciosa.* The use of enzymes with potential strictosidine reductase, strictosidine glucosidase, geissoschizine synthase, hydroxylase, and O-methyltransferase activity are described herein, for example, MsSTR1, MsSGD, MsGSS1, homologs of T16H, T16OMT, LAOMT, and related sequences.

*In vitro* catalysis systems, designed to utilize these enzymes and thereby produce substantially pure monoterpene indole alkaloids, such as strictosidine, strictosidine aglycone, 4,21-dehydrogeissoschizine, geissoschizine, corynantheidine, 9-hydroxycorynantheidine, mitragynine, 7-hydroxymitragynine, speciociliatine, speciogynine, and paynantheine, are described.

Like mitragynine, these and other monoterpene indole alkaloids may find use in compositions and methods to treat pain and to reduce dependence on opioids and opiates.

### DEFINITIONS

Unless defined otherwise, technical and scientific terms used herein have the same meaning as commonly understood by one of ordinary skill in the art to which this invention belongs. See, e.g. Singleton et al., Dictionary of Microbiology and Molecular Biology 2nd ed., J. Wiley & Sons (New York, N.Y. 1994); Sambrook et al., Molecular Cloning. A Laboratory Manual, Cold Springs Harbor Press (Cold Springs Harbor, NY 1989), each of which is incorporated herein by reference. For the purposes of the present application, the following terms are defined below.

As used herein, the term "monoterpene indole alkaloid" refers to compounds containing a bicyclic structure of a benzene ring fused to a five-membered pyrrole ring. These compounds may be of natural or synthetic origin. In some examples, such compounds have a chemical structure derived from strictosidine. Some examples of monoterpene indole alkaloids include those typically found in *M*. *speciosa,* such as geissoschizine, ajmalicine, yohimbine, reserpine, sarpagin, mitragynine, cathenamine, catharanthine, tabersonine, vindolin, vincamine. In a particular aspect, these embodiments refer to the corynanthe-type of monoterpenoid indol alkaloids, for example, strictosidine, strictosidine aglycone, geissoschizine, corynantheidine, 9-hydroxycorynantheidine, mitragynine, 7-hydroxymitragynine, speciociliatine, speciogynine, and paynantheine.

The following structures of Geissoschizine and Ajmalicine are examples of monoterpene indole alkaloids. The numbering in the ring structure of Geissoschizine indicates positions where potential modifications such as hydroxylations and subsequent methylations can occur. As studied by Takayama (Takayama, 2004), the position of such modifications can affect the bioactivity of the molecule. 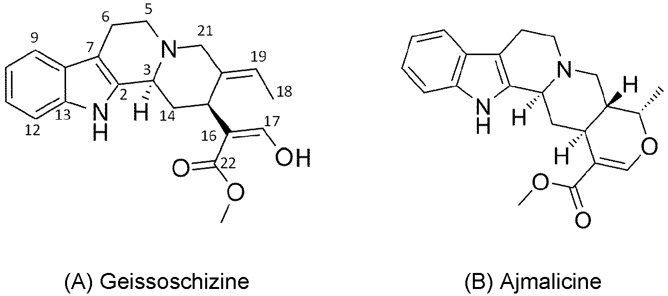 Examples of corynanthe-type monoterpenoid indole alkaloids

As used herein, the term "variants" refers to variants of the sequences described herein. The term "variants" refers to biologically active sequences that have a peptide sequence that differs from the sequence of a native or wild-type sequence, by virtue of an insertion, deletion, modification, and/or substitution of one or more amino acids within the native sequence. Such variants have less than 100% sequence identity with a native sequence. Ordinarily, however, a biologically active variant will have an amino acid sequence with at least about 70% sequence identity with the sequence of a corresponding naturally occurring sequence, typically at least about 75%, more typically at least about 80%, even more typically at least about 85%, even more typically at least about 90%, and even more typically of at least about 95%, 96%, 97%, 98%, or 99% sequence identity. The polypeptide variants can include fragments of any length that retain the biological activity of the corresponding native sequence. Variants can also include sequences wherein one or more amino acids are added at either end of, or within, a native sequence. Variants can also include sequences where a number of amino acids are deleted and optionally substituted by one or more different amino acids.

As used herein, the term "treatment" or "therapy" refers to an approach for obtaining beneficial or desired clinical results. For the purposes described herein, beneficial or desired clinical results include, but are not limited to, alleviation of symptoms, diminishment of the extent of disease, stabilized (i.e., not worsening) state of disease, delay or slowing of disease progression, amelioration or palliation of the disease state, and remission (whether partial or total), whether detectable or undetectable. "Treatment" and "therapy" can also mean prolonging survival as compared to expected survival if not receiving treatment or therapy. Thus, "treatment" or "therapy" is an intervention performed to alter the pathology of a disorder. Specifically, the treatment or therapy may directly prevent, slow down or otherwise decrease a disorder such as pain, or may render the pain more susceptible to treatment or therapy by other therapeutic agents.

The terms "therapeutically effective amount", "effective amount" or "sufficient amount" mean a quantity sufficient, when administered to a subject, including a mammal, for example, a human, to achieve the beneficial or desired result, for example, an amount effective to treat pain. Effective amounts of the alkaloid compounds described herein may vary according to factors such as the disease state, age, sex, and weight of the subject. Dosage or treatment regimens may be adjusted to provide the optimum therapeutic response, as is understood by a skilled person.

Likewise, an "effective amount" of the indole alkaloid compounds described herein refers to an amount sufficient to function as desired, such as to treat pain.

Administration "in combination with" one or more further therapeutic agents includes simultaneous (concurrent) and consecutive administration in any order.

The term "pharmaceutically acceptable" means that the compound or combination of compounds is compatible with the remaining ingredients of a formulation for pharmaceutical use and that it is generally safe for administering to humans according to established governmental standards, including those promulgated by the United States Food and Drug Administration.

"Carriers" as used herein include cosmetically or pharmaceutically acceptable carriers, excipients, or stabilizers that are non-toxic to the cell or subject being exposed thereto at the dosages and concentrations employed. Often the pharmaceutically acceptable carrier is an aqueous pH buffered solution. Examples of pharmacologically acceptable carriers include buffers such as phosphate, citrate, and other organic acids; antioxidants including ascorbic acid; low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, and dextrins; chelating agents such as EDTA; sugar alcohols such as mannitol and sorbitol; salt-forming counterions such as sodium; and/or non-ionic surfactants such as TWEEN^{™}, polyethylene glycol (PEG), and PLURONICS^{™}.

In understanding the scope of the present application, the articles "a", "an", "the", and "said" preceding an element are intended to mean that there are one or more of the elements. Additionally, the term "comprising" and its derivatives, as used herein, are intended to be open-ended terms that specify the presence of the stated features, elements, components, groups, integers, and/or steps, but do not exclude the presence of other unstated features, elements, components, groups, integers and/or steps. The foregoing also applies to words having similar meanings such as the terms, "including", "having", and their derivatives.

It will be understood that any embodiments described as "comprising" certain components may also "consist of" or "consist essentially of," whereas "consisting of" has a closed-ended or restrictive meaning and "consisting essentially of" means including the components specified but excluding other components except for materials present as impurities, unavoidable materials present as a result of processes used to provide the components, and components added for a purpose other than achieving the technical effect of the invention. For example, a composition defined using the phrase "consisting essentially of" encompasses any known pharmaceutically acceptable additive, excipient, diluent, carrier, and the like. In some embodiments, a composition consisting essentially of a set of components will comprise less than 5% by weight, typically less than 3% by weight, more typically less than 1% by weight of non-specified components.

In addition, all ranges given herein include the end of the ranges and also any intermediate-range points, whether explicitly stated or not.

Finally, terms of degree such as "substantially", "about" and "approximately" as used herein mean a reasonable amount of deviation of the modified term such that the end result is not significantly changed. These terms of degree should be construed as including a deviation of at least ±5% of the modified term if this deviation would not negate the meaning of the word it modifies.

### Polypeptides

Described herein, in aspects, are polypeptides encoding enzymes. These polypeptides are typically derived from plants such as *Mitragyna speciosa* and find use in producing monoterpene indole alkaloids with medicinal activity and/or precursors to such compounds. For example, described herein are polypeptides comprising one or more of the following sequences, variants thereof, or fragments of the polypeptides or variants. Variants can be of natural origin such as orthologous sequences from related plant species, or of non-natural origin obtained synthetically or by mutations. The following sequences, are shown in attached Sequence Listing.
- SEQ IDs No: 1-13 - polypeptide sequences orthologous to strictosidine-O-beta-D-glucosidase (SGD)
- SEQ IDs No: 14-22 - polypeptide sequences orthologous to geissoschizine synthase (GSS/THAS)
- SEQ IDs No: 23-54 - polypeptide sequences homologous to tabersonine 16-hydroxylase (T16H)
- SEQ IDs No: 55-61 - polypeptide sequences homologous to tabersonine 16-O-methyltransferase (T16OMT)
- SEQ IDs No: 62-65 - polypeptide sequences homologous to loganic acid O-methyltransferase (LAOMT)

The variants of the polypeptides described herein may have any degree of sequence identity to the polypeptides, provided they retain some degree of native activity of the corresponding native sequence, for example strictosidine reductase, strictosidine glucosidase, geissoschizine synthase, hydroxylase, or O-methyltransferase activity. For example, the variants typically have at least about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 99.5% sequence identity.

Likewise, the fragments of the polypeptides or variants described herein may have any length, provided they retain some degree of native activity, for example, strictosidine reductase, strictosidine glucosidase, geissoschizine synthase, hydroxylase, or O-methyltransferase activity. For example, the fragments may be missing about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 125, about 150, about 175, about 200, or about 250 amino acid residues as compared to the polypeptide in question.

The polypeptides, variants, and fragments described herein may also be fused to other polypeptides and could, therefore comprise additional amino acid residues, such as about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 125, about 150, about 175, about 200, about 250, about 300, about 400, about 500, about 600, about 700, about 800, about 900, or about 1000 or more additional amino acids.

The polypeptides described herein may comprise, consist of, or consist essentially of the sequences of SEQ ID NO: 1-65, and typically encode an enzyme.

The polypeptides described herein are typically expressed in a host cell or organism, such as a bacterium, a yeast, an alga, a fungus, or a plant, including single cells and cell cultures of any thereof for enzymatically acting on a molecule present in the host cell or organism or its cell culture medium. The polypeptides described herein may instead be used in a cell-free system for acting on a molecule present in the system.

In embodiments, the hosts described herein endogenously express and/or are engineered to express at least one nucleic acid coding for an enzyme with strictosidine reductase, strictosidine glucosidase, geissoschizine synthase, hydroxylase, or O-methyltransferase activity or variants thereof to produce a monoterpene indole alkaloid, such as strictosidine, strictosidine aglycone, geissoschizine, corynantheidine, 9-hydroxycorynantheidine, mitragynine, 7-hydroxymitragynine, speciociliatine, speciogynine, or paynantheine, or variants thereof.

Host cells described herein can be any cell capable of producing at least one protein described herein and include bacterial, fungal (including yeast), animal, algal, and plant cells. The cells may be prokaryotic or eukaryotic. Typical host cells are bacterial, yeast, algal, and plant cells. In a typical embodiment, the plant cell is a seed cell, in particular, a cell in a cotyledon or endosperm of a seed. In one embodiment, the cell is a bacterial cell. An example of a bacterial cell useful as a host cell of the present invention is *Escherichia coli, Synechococcus* spp. (also known as *Synechocystis* spp.), for example, *Synechococcus elongatus.* Examples of algal cells useful as host cells of the present invention include, for example, *Chlorella* sp., *Chlamydomonas* sp. (for example, *Chlamydomonas reinhardtii*), *Dunaliella* sp., and *Haematococcus* sp. It will be understood that some host cells may not be able to produce an alkaloid because they lack the necessary precursors but can be used as host cells to produce the polypeptides described herein. Thus, it will be understood that the polypeptides described herein can be expressed in a variety of expression host cells e.g., bacteria, yeasts, plant cells, algal cells, or cell-free expression systems. In one embodiment, described herein are expression vectors comprising the coding DNA sequence for the polypeptides described herein for the expression and purification of the recombinant polypeptide produced from a protein expression system using host cells selected from, e.g., bacteria, mammalian, insect, yeast, or plant cells. In some embodiments, the nucleic acid can be sub-cloned into a recombinant expression vector that is appropriate for the expression of fusion polypeptide in bacteria, mammalian, yeast, or plant cells or a cell-free expression system such as the wheat germ cell-free expression system or a rabbit reticulocyte expression system.

In some embodiments, cell-free systems can include *in vitro* enzymatic reactions performed in tubes, columns, chips, or any other solid support or surface where a polypeptide described herein is present in solution or immobilized in a resin or another solid support matrix. A range of reversible physical adsorption and ionic linkages, to irreversible stable covalent bonds exist to produce immobilized enzymes.

Thus, in embodiments, the host cells or cell-free systems described herein are suitable for producing a substantially pure polypeptide and/or substantially pure indole alkaloid in the presence of at least an enzyme such as those represented by SEQ ID: 1-65 (or fragments or variants thereof).

In embodiments, the cell-free systems can include a previous modification step such as a nucleic acid coding for secologanin synthase to produce secologanine and for tryptophan decarboxylase to produce tryptamine which can then both be used by a polypeptide described herein to synthesize a substantially pure strictosidine.

In embodiments, the production of indole alkaloid molecules with the polypeptides described herein are carried out following a unique order of reactions, but such polypeptides can be used with different substrates in the described pathway or similar molecules to produce the described indole alkaloids or variants.

In embodiments, the enzymatic reactions catalyzed by these polypeptides can also be combined employing organic chemistry synthesis methods.

Also described herein are compositions comprising an indole alkaloid obtainable or obtained by one of the methods as disclosed above, and the use of the said composition as a medicinal agent, such as an analgesic agent, for pharmacological purposes.

Provided herein are compositions comprising substantially pure indole alkaloids, which are, for example, at least about 95%, about 96%, about 97%, about 98%, about 99%, about 99.5%, or about 99.9% pure.

The compositions comprising an indole alkaloid described herein may be formulated for use by a subject, such as a mammal, including a human. Such compositions may comprise about 0.00001% to about 99% by weight of the active compound(s) and any range there-in-between. For example, typical doses may comprise from about 0.1 µg to about 100 µg of the molecules described herein per 300 mg dose, such as about 0.5 µg, about 1 µg, about 2 µg, about 3 µg, about 4 µg, about 5 µg, about 6 µg, about 7 µg, about 8 µg, about 9 µg, about 10 µg, about 25 µg, about 50 µg, or about 75 µg per 300 mg dose, such as from about 0.1 µg to about 10 µg, or from about 1 µg to about 5 µg, or from about 1 µg to about 2 µg per 300 mg dose (and all related increments and percentages by weight).

The alkaloid molecules described herein may be used in any suitable amount, but are typically provided in doses comprising from about 1 to about 10000 ng/kg, such as from about 1 to about 1000, about 1 to about 500, about 10 to about 250, or about 50 to about 100 ng/kg, such as about 1, about 10, about 25, about 50, about 75, about 100, about 150, about 200, about 250, about 300, or about 500 ng/kg. Similar amounts, higher amounts, or lower amounts could be used for administration.

The indole alkaloid molecules described herein may be administered over a period of hours, days, weeks, or months, depending on several factors, including the severity and type of pain or other condition being treated, whether a recurrence is considered likely, or to prevent pain or other condition, etc. The administration may be constant, e.g., constant infusion over a period of hours, days, weeks, months, etc. Alternatively, the administration may be intermittent, e.g., the molecules may be administered once a day over a period of days, once an hour over a period of hours, or any other such schedule as deemed suitable.

The compositions described herein can be prepared by per se known methods for the preparation of pharmaceutically or cosmetically acceptable compositions which can be administered to subjects, such that an effective quantity of the active substance is combined in a mixture with a pharmaceutically acceptable vehicle. Suitable vehicles are described, for example, in "Handbook of Pharmaceutical Additives" (compiled by Michael and Irene Ash, Gower Publishing Limited, Aldershot, England (1995)). On this basis, the compositions include, albeit not exclusively, solutions of the substances in association with one or more pharmaceutically acceptable vehicles or diluents, and may be contained in buffered solutions with a suitable pH and/or be iso-osmotic with physiological fluids.

Pharmaceutically acceptable carriers are well known to those skilled in the art and include, for example, sterile saline, lactose, sucrose, calcium phosphate, gelatin, dextrin, agar, pectin, peanut oil, olive oil, sesame oil, cannabis oil, and water. Furthermore, the composition may comprise one or more stabilizers such as, for example, carbohydrates including sorbitol, mannitol, starch, sucrose, dextrin, and glucose, proteins such as albumin or casein, and buffers like alkaline phosphates.

The indole alkaloid molecules described herein can, in embodiments, be administered for example, by parenteral, intravenous, subcutaneous, intradermal, intramuscular, intracranial, intraorbital, ophthalmic, intraventricular, intracapsular, intraspinal, intracisternal, intraperitoneal, intranasal, intrarectal, intravaginal, aerosol, oral, topical, or transdermal administration. Typically, the compositions of the invention are administered orally.

It is understood by one of skill in the art that the produced molecules described herein can be used in conjunction with known therapies for prevention and/or treatment of pain in subjects. Similarly, the produced modified molecules described herein can be combined with one or more other pharmaceutical or natural health products, such as cannabinoids, terpenes, or other natural or synthetic compounds. The produced molecules described herein may, in embodiments, be administered in combination, concurrently or sequentially, with conventional treatments for pain, including non-steroidal anti-inflammatory drugs, for example. The indole alkaloid molecules described herein may be formulated together with such conventional treatments when appropriate. Other uses of these alkaloid molecules can be found due to their anticipated antidepressant, anxiolytic, antipsychotic, antihypertensive, and antipyretic activities, or for the treatment or withdrawal from opioid and alcohol dependence and abuse.

Further embodiments of the invention are set out in numbered Embodiments 1 to 41 below.
1. A polypeptide encoding a strictosidine glucosidase that removes the glucose moiety from strictosidine to produce strictosidine aglycone.
2. The polypeptide of Embodiment 1, wherein the strictosidine glucosidase is from *Mitragyna speciosa.*
3. The polypeptide of Embodiment 2, comprising or consisting of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 1-13, or a fragment of any thereof.
4. The polypeptide of any one of Embodiments 1 to 3, wherein the polypeptide produces strictosidine-aglycone when reacted under suitable conditions in the presence of strictosidine.
5. A polypeptide encoding a geissoschizine synthase that opens the tetrahydropyran ring of strictosidine aglycone to produce geissoschizine.
6. The polypeptide from Embodiment 5, wherein the geissoschizine synthase is from *Mitragyna speciosa.*
7. The polypeptide from Embodiment 6, comprising or consisting of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 14-22, or a fragment of any thereof.
8. The polypeptide of any of Embodiment 5 to 7, wherein the polypeptide produces geissoschizine when reacted under suitable conditions in the presence of strictosidine aglycone.
9. A polypeptide encoding a hydroxylase that hydroxylates the indole ring of coηnantheidine at the C9 position to produce 9-hydroxycorynantheidine.
10. A polypeptide encoding a hydroxylase that hydroxylates the indole ring of mitragynine at the C7 position to produce 7-hydroxymitragynine.
11. The polypeptide of Embodiments 9 and 10, wherein the hydroxylase is from *Mitragyna speciosa.*
12. The polypeptide of any one of Embodiments 9 to 11, comprising or consisting of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 23-54, or a fragment of any thereof.
13. The polypeptide of any one of Embodiments 9, 11 and 12, wherein the polypeptide produces a 9-hydroxy-indole alkaloid, such as 9-hydroxycorynantheidine.
14. The polypeptide of any one of Embodiments 10 to 12, wherein the polypeptide produces 7-hydroxymitragynine when reacted under suitable conditions in the presence of mitragynine.
15. A polypeptide encoding an O-methyltransferase that methylates the hydroxyl group at the C9 position of 9-hydroxycorynantheidine to produce mitragynine.
16. The polypeptide of Embodiment 15, wherein the O-methyltransferase is from *Mitragyna speciosa.*
17. The polypeptide of any one of Embodiments 15 and 16, comprising or consisting of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 55-65, or a fragment of any thereof.
18. The polypeptide of any one of Embodiments 15 to 17, wherein the polypeptide produces mitragynine when reacted under suitable conditions in the presence of 9-hydroxycorynantheidine.
19. The polypeptide of any one of Embodiments 1 to 18, wherein the polypeptide comprises up to about 100, about 150, about 200, about 250, about 300, about 350, about 360, about 370, about 380, about 390, about 400, about 410, about 420, about 430, about 440, about 450, or about 500 amino acids.
20. The polypeptide of any one of Embodiments 1 to 19, wherein the polypeptide is synthetic.
21. The polypeptide of any one of Embodiments 1 to 20, wherein the polypeptide is recombinant.
22. A nucleic acid encoding the polypeptide of any one of Embodiments 1 to 21.
23. The nucleic acid of Embodiment 22, wherein the nucleic acid is cDNA.
24. A vector comprising the nucleic acid of Embodiment 22 or 23.
25. A host cell comprising the vector of Embodiment 24.
26. A host cell expressing the polypeptide of any one of Embodiments 1 to 21.
27. The host cell of Embodiment 25 or 26, wherein the host cell is a bacterial cell (e.g., E. coli or Agrobacterium tumefaciens), a yeast cell (e.g., S. cerevisiae), an algal cell, or a plant cell (e.g., Nicotiana spp.).
28. The host cell of any one of Embodiments 25 to 27, in combination with an indole alkaloid precursor.
29. The host cell of Embodiment 28, wherein the indole alkaloid precursor is provided in the host cell culture medium.
30. The host cell of Embodiment 28 or 29, wherein the indole alkaloid precursor is expressed by the host cell.
31. An expression system comprising the polypeptide of any one of Embodiments 1 to 21; the nucleic acid of Embodiment 22 or 23, the vector of Embodiment 24; or the host cell of any one of Embodiments 25 to 30.
32. A system for modifying an indole alkaloid, the system comprising the polypeptide of any one of Embodiments 1 to 21.
33. The system of Embodiment 32, wherein the system is cell-free.
34. A method for modifying an indole alkaloid, wherein the method comprises contacting the indole alkaloid with the polypeptide of any one of Embodiments 1 to 21.
35. The method of Embodiment 34, wherein the method is a recombinant method comprising expressing a polypeptide from any one of Embodiments 1 to 21 in a cell in the presence of an indole alkaloid precursor.
36. The method of Embodiment 34 or 35, comprising a single or combined enzymatic reaction steps.
37. A method of producing strictosidine aglycone, 4,21-dehydrogeissoschizine, geissoschizine, corynantheidine, 9-hydroxycorynantheidine, mitragynine, 7-hydroxymitragynine, speciociliatine, speciogynine, and paynantheine, the method comprising carrying out combined enzymatic reactions with the polypeptides from any one of Embodiments 1 to 21.
38. The indole alkaloid of Embodiment 37, wherein the indole alkaloid is substantially pure, for example, at least about 95%, about 96%, about 97%, about 98%, about 99%, about 99.5%, or about 99.9% pure.
39. A pharmaceutical composition comprising the indole alkaloid of Embodiments 37 or 38 and at least one pharmaceutically acceptable carrier.
40. A natural health product comprising the indole alkaloid of Embodiments 37 or 38, such as a supplement, beverage, or food.
41. Use of the indole alkaloid of Embodiments 37 or 38 in a pharmaceutical, or natural health product.

The above disclosure generally describes the present invention. A more complete understanding can be obtained by reference to the following specific Examples. These Examples are described solely for purposes of illustration and are not intended to limit the scope of the invention. Changes in form and substitution of equivalents are contemplated as circumstances may suggest or render expedient. Although specific terms have been employed herein, such terms are intended in a descriptive sense and not for purposes of limitation.

### Methods

### Plant Material and Growth Conditions.

*Mitragyna speciosa* (Korth.) Havil plants (Malay and Rifat varieties) can be maintained in growth chambers with a 16-hour light photoperiod (200 µmol m⁻²s⁻¹; mixed cool white and incandescent bulbs), a day/night temperature regime of 28°C/26°C, and a constant relative humidity of 80%. Plants are grown in a 2:1:1 (v/v) mixture of coco coir (Millennium soils Coir):perlite (Therm-o-rock East Inc.):turface (Turface Athletics) before harvesting material. The plant material was identified and authenticated by Dr. Carole Ann Lacroix and a voucher specimen (No. 102033) was deposited at the Ontario Agricultural College Herbarium in Guelph, Ontario, Canada.

### Mitragyna transcript sequences.

In typical aspects, total mRNA is extracted from young and mature leaves using the RNeasy Plant mRNA extraction kit (Qiagen). Quality control, normalization, mRNA Seq library construction, and *de novo* transcriptome assembly were performed by Macrogen Inc. (macrogen.com; Seoul, Korea) in order to reconstruct transcript sequences without a reference genome. Transcripts are represented by assembled contigs and then merged using the Trinity program, and then clustered into non-redundant transcripts or unigenes using CD-HIT-EST program with a minimum length of 200 bp. The obtained unigenes can then be used for the subsequent annotation, ORF (open reading frame) prediction, and expression analysis. ORF prediction for the unigenes is performed using TransDecoder program to identify candidate coding regions.

### Identification of enzymes for indole alkaloid biosynthesis in Mitragyna.

Nucleotide sequences encoding for putative enzymes required for the different steps for the biosynthesis of mitragynine and related alkaloids can be obtained by searching for homologous sequences in the *de novo* transcriptome analysis described above using sequences of other plant species known to participate in alkaloid metabolism. The tblastn queries were perform with *Catharanthus roseus'* strictosidine-O-beta-D-glucosidase (B8PRP4), geissoschizine synthase (crGS1spIW8JWW7), tabersonine 16-hydroxylase 1 (crT16H1sp|P98183), tabersonine 16-hydroxylase 2 (crT16H2sp|U5HKE8), tabersonine 16-O-methyltransferase (cr16OMTsp|B0EXJ8), and loganic acid O-methyltransferase (crLAMTsp|B2KPR3); strictosidine synthase (rvSTR_sp|P68174) from *Rauvolfia mannii,* and strictosidine-O-beta-D-glucosidase (rsSGD_sp|Q8GU20) from *Rauvolfia serpentina.* In addition, other orthologous sequences were obtained with tryptophan decarboxylase (msTDC_tr|G5DF84) and secologanin synthase (msSLS3_tr|H9MZK1) from *Mitragyna speciosa.* When possible, the sequences were then matched against the Mitragyna genome contig database, via BLASTn searches, to retrieve the full-length open reading frames for the selected sequences. The *in-silico* assembled amino acid sequences from these gene sequences were then used to construct phylogenetic relationships using the MEGA software package (version 6.0) by the neighbor-joining method with bootstrap analysis of 1000 replicates.

### Expression of recombinant in E. coli.

In typical aspects, open reading frames encoding the selected candidate enzymes are synthesized commercially. Each cDNA is amplified by PCR and then inserted into an expression vector system (for example, Novagen's pET28) which introduces an N-terminal 6x His tag to the coding sequence. The construct is then introduced into a bacterial host (for example E. *coli* BL21-CodonPlus (DE3)-RIPL cells). Bacterial cells expressing a recombinant enzyme are cultured in LB media for several hours in the presence of IPTG to induce recombinant protein expression. The bacterial cells are collected by centrifugation, re-suspended, and then disrupted by sonication. Crude protein extracts are applied and purified with a Ni²⁺ affinity matrix (for example a HisTrap FF column). Afterward, the enzyme is eluted with high concentrations (250 to 400 uM) of imidazole, and then immediately desalted on PD-10 columns equilibrated in a suitable buffer. Protein concentration is determined by Bradford Assay using BSA as a standard. The purified protein can then be frozen before use.

### Chemicals and reagents.

Authentic alkaloid standards can be purchased from specialized chemical companies: Ajmalicine (Sigma-Aldrich) and mitragynine, 7-hydroxymitragynine, paynantheine, speciogynine, mitraphylline, speciociliatine, tryptamine, secologanin, strictosidine-aglycone, and 19Z-geissoschizine (Cayman Chemicals). Other chemicals used in enzymatic reactions can be procured from Sigma-Aldrich. These external alkaloid standards can be used for quantification based on the peak area revealed by the HPLC analyses.

### Mass spectrometry analysis of enzymatic reaction products.

Generally, samples are evaporated under nitrogen and then re-suspended in methanol prior to liquid chromatography-mass spectrometry (LC-MS) analysis (for example HPLC (high performance liquid chromatography) interfaced with a Q-TOF (time-of-flight) mass spectrometer). Typically, samples can be resolved with a reverse-phase column and eluted over a gradient from 45% to 95% methanol with 0.1% formic acid (v/v), or with 1:1 water and acetonitrile as solvents, both with 0.1 % formic acid, followed by 100% methanol for 10-20 min. The eluted products are detected by absorption at the 210-350 nm range and quantified relative to authentic standards based on the peak area of the HPLC analysis. During the LC-MS analysis, the mass-to-charge ratio is typically scanned across the m/z range of 100-3000 m/z in an extended dynamic range positive-ion MS mode. Chromatograms can be analyzed by using a software that compares MS patterns from standard libraries or known standards used in the laboratory. Fragmentation patterns of the various parent (molecular) ions obtained using collision energies of 5 to 20 V from the recovered peak products (modified compounds) are usually also compared with fragmentation patterns of standard molecules.

In typical aspects, nuclear magnetic resonance (NMR) spectroscopy, is used as a preeminent technique for determining the structure of the indole alkaloids that can be obtained (for example for determining the position of double bonds, hydroxyl, or methyl groups). After the enzymatic reaction products from the enzymatic assays are resolved by HPLC, the compounds are eluted and subsequently collected. Usually, approximately 0.5 mg of each compound is evaporated to dryness under N₂ gas, resuspended in acetone-d6, and analyzed using ¹H and ¹³C NMR. NMR spectra are collected on a spectrometer (for example a Bruker AVANCE III 600 MHz equipped with a 5 mm TCI cryoprobe).

This or a similar in vitro cell-free system described herein can be suitable for producing a substantially pure indole alkaloid in the presence of at least an enzyme described herein.

### Examples

### Example 1

This example describes a general method for analyzing alkaloids from *Mitragyna speciosa,* which also validates the process used for the identification of enzymatic products generated below. About 100 g of dried leaf material was extracted with 2 L of an acetic acid solution (0.5 M) at 80°C for 30 min. The crude extract was filtered and then passed through a 60 ml column containing polyvinylpyrrolidone (PVPP, 110 µm particle size) to remove polyphenolic compounds and then sequentially chromatographed over 50 ml of Diaion HP-20 resin (Supelco) equilibrated with distilled water and the reversed-phase column was then washed with 20% (v/v) methanol before elution with 100% methanol followed by methanol/ethyl acetate (50:50 v/v). The recovered alkaloid fractions were pooled and reduced to 200 ml on a rotary evaporator at 70°C. The isolate was then loaded onto an ion-exchange resin (AmberChrom 50WX2, 200-400) and washed with 500 ml of acetic acid in ethanol (0.025 M), followed by 250 ml of 100% ethanol. Alkaloids were eluted with 340 ml of 2.8 M ammonium hydroxide in ethanol and then brought to a 150 ml final volume. This purified alkaloid extract was subjected to phase separation with chloroform (300 ml). The organic layer was extracted and reduced to dryness, in vacuo, and resuspended in 10 ml of 0.2 M HCl. After complete resuspension, the pH was adjusted to 5.0. Alkaloids fractions were analyzed using an Agilent 1260 Infinity liquid chromatography system equipped with a reversed-phase Kinetex EVO C18 100Å column (150 × 4.6 mm, 5 µm). Separation of the alkaloids was achieved using a binary gradient with ammonium bicarbonate buffer (5 mM pH 9.5; A) and acetonitrile (B), starting with 70% solvent A transitioning to 70% solvent B for 18 min at a flow rate of 1.5 ml/min. Alkaloids were quantified at 226 nm. Chemical structures of common alkaloids detected in kratom are shown in **FIGURE 1A** and an example of an HPLC chromatogram illustrating the alkaloid profile obtained with a purified crude extract is presented in **FIGURE 1B****,** where different peaks (top panel) can be compared with standards (bottom panel). For example, Mitragynine elutes at 14.4 min.

To verify the identity of the molecules, the alkaloid fractions were collected and approximately 0.5 mg of each compound were evaporated to dryness, resuspended in deuterated chloroform, and analyzed using ¹H NMR. NMR spectra were collected on a Bruker AVANCE III 600 MHz spectrometer equipped with a 5 mm TCI cryoprobe. According to their chemical structure, and to what has been described in *Catharanthus resets* for the synthesis of alkaloids (e.g. cathenamine), a biosynthetic pathway of monoterpene indole alkaloids in *M*. *speciosa* is proposed **(****FIGURE 2****).** From it, enzyme-encoding homologous sequences were searched in the transcriptome and potential candidates were identified.

The following examples describe general methods for using polypeptide sequences from *Mitragyna speciosa* to produce monoterpene indole alkaloids and its intermediaries.

### Example 2

This example describes a general method for using strictosidine reductase polypeptide sequences from *Mitragyna speciosa* to produce strictosidine from secologanine and tryptamine **(****FIGURE 3A****).** A synthetic cDNA sequence of the MsSTR-1 sequence was sub-cloned in the pET28a vector system (Novagen) which introduced an N-terminal 6x His tag to the coding sequence and then the construct was introduced into *E*. *coli* BL21-CodonPlus (DE3)-RIPL cells. Bacterial cells expressing recombinant MsSTR-1 were used to produce recombinant MsSTR-1 protein as described in the Methods above, and stored at -80°C before use.

The enzymatic assays with recombinant enzyme MsSTR-1 consisted of a total volume of 100 µL of 2 mM of secologanin, 1 mM tryptamine, and 10 µg of recombinant STR enzyme in 100 mM NaH₂PO₄ buffer pH 6.8 at 30°C for 24 h. A control reaction was performed with boiled enzyme. Reactions were terminated by boiling for 2 min, centrifuged for 5 min and 20 µL was injected on an HPLC. Strictosidine was analyzed using an Agilent 1260 Infinity liquid chromatography system equipped with a reverse phase Waters Spherisorb 5 µM (ODS2 4.6 x 250 mm) column. The chromatographic separation of tryptamine, secologanin and strictosidine was achieved using a binary gradient with (0.1% TFA; A) and (acetonitrile; B) gradient at a flow rate of 1 mL/min. The amount of solvent A in the mobile phase under the starting conditions was 68% and decreased to 30% until 16 min. Strictosidine and tryptamine were detected by absorption at 280 nm, and the amount of strictosidine produced was reported in equivalents of tryptamine. The HPLC chromatogram of the product showed a major peak corresponding to stroctisidine according to the eluted time of the standard **(****FIGURE 3B****).** The peak was collected and processed for mass spectrometry analysis.

Samples were evaporated under nitrogen and then re-suspended in methanol prior to liquid chromatography-mass spectrometry (LC-MS) analysis performed on an Agilent 1200 HPLC interfaced with an Agilent UHD 6530 Q-TOF mass spectrometer. A C18 cartridge column (Agilent Rapid Resolution 2.1 × 30 mm, 3.5 µm) was used at 30°C with 1:1 water and acetonitrile as solvents, both with 0.1 % formic acid. The flow rate was maintained at 0.4 ml/min. The mass spectrometer electrospray capillary voltage was maintained at 4.0 Kv and the drying gas temperature at 250°C with a flow rate of 8 L/min. Nitrogen was used as both nebulizing, drying gas, and collision-induced dissociation gas. The instrument was externally calibrated with the ESI TuneMix (Agilent). Chromatograms were analyzed within Agilent Qualitative Analysis software B 08.0. Fragmentation patterns of the various parent (molecular) ions were obtained using collision energies of 5, 10 and 20 V. HPLC profile and the mass spectral fragmentation pattern obtained from the recovered peak product of the assay **(****FIGURE 3C****)** corresponded to the mass spectra observed with the strictosidine standard.

### Example 3

This example describes a general method for using geissoschizine synthase polypeptide sequences from *Mitragyna speciosa* to produce geissoschizine from stictosidine aglycone **(****FIGURE 4A****).** A synthetic cDNA sequence of the MsGSS-1 sequence was sub-cloned in the pET28a vector system (Novagen) which introduced an N-terminal 6x His tag to the coding sequence and then the construct was introduced into E. *coli* BL21-CodonPlus (DE3)-RIPL cells. Bacterial cells expressing recombinant MsGSS-1 were used to produce a recombinant MsGSS-1 protein as described in the Methods above, and stored at -80°C before use.

Enzymatic reactions consisted in a total volume of 100 µL of 135 µM of strictosidine-aglycone, 200 µM of NADPH and 2 - 8 µg of recombinant MsGSS-1 enzyme in 20 mM Tris-HCl buffer pH 7.5 at 30°C for 1 h and 6 h. A control reaction was performed with boiled enzyme. Reactions were terminated by boiling for 2 min, centrifuged for 5 min and extracted twice with ethyl-acetate. After drying under nitrogen gas, samples were resuspended in 60 µl methanol and 20 µl were injected in the HPLC. Geissoschizine was analyzed using an Agilent 1260 Infinity liquid chromatography system equipped with a reverse phase column Kinetex EVO C18 100Å (150 × 4.6 mm, 5 µm). Chromatographic separation of geissoschizine was achieved using a binary gradient with ammonium bicarbonate buffer (5 mM, pH 9.5; A) and acetonitrile (B), starting with 70% solvent A transitioning to 70% solvent B over the course of 17 minutes at a flow rate of 1.5 mL/min. 19Z-geissoschizine was quantified at 226 nm. The chromatograms of the products showed the same peak **(****FIGURE 4B****),** and peak areas of all five reactions (control, 2 µg protein 1 h, 2 µg protein 6 h, 8 µg protein 1 h, and 8 µg protein 6 h) were calculated **(****FIGURE 4C****).** Representative peaks were collected and processed for mass spectrometry analysis. For this, samples were evaporated under nitrogen and then re-suspended in methanol before liquid chromatography-mass spectrometry (LC-MS) analysis was performed on an Agilent 1200 HPLC interfaced with an Agilent UHD 6530 Q-TOF mass spectrometer, using the conditions described in the previous example. The chromatogram of the reaction product showed three peaks that eluted at 1.8, 2.5, and 12.9 min in samples that had the 'live' enzyme and not in samples with boiled enzyme **(****FIGURE 5A****).** HPLC profile and the mass spectral fragmentation pattern obtained from the recovered peak product at 12.9 min **(****FIGURE 5B****)** corresponded to the mass spectra of a geissoschizine standard.

The above disclosure generally describes the present invention. Although specific terms have been employed herein, such terms are intended in a descriptive sense and not for purposes of limitation.

All publications, patents, and patent applications cited herein are incorporated by reference in their entirety to the same extent as if each publication, patent, or patent application was specifically and individually indicated to be incorporated by reference in its entirety.

Although preferred embodiments of the invention have been described herein in detail, it will be understood by those skilled in the art that variations may be made thereto without departing from the spirit of the invention or the scope of the appended claims.

A sequence listing is being filed herewith, the sequence listing is incorporated herein by reference in its entirety.

### References

Adkins JE, Boyer EW, McCurdy CR. (2011) Mitragyna speciosa, a psychoactive tree from Southeast Asia with opioid activity. Curr Top Med Chem. 11: 1165-1175.
Avery BA, Boddu SP, Sharma A, Furr EB, Leon F, Cutler SJ, et al. (2019) Comparative pharmacokinetics of mitragynine after oral administration of Mitragyna speciosa (kratom) leaf extracts in rats. Planta Med. 2019 85: 340-346.
Center of Disease Control. Opioid Crisis, Understanding the Epidemic. https://www.cdc.gov/opioids/basics/epidemic.html. Accessed February 07, 2022
Coe MA, Pillitteri JL, Sembower MA, Gerlach KK, Henningfield JE (2019) Kratom as a substitute for opioids: Results from an online survey. Drug Alcohol Depend. 202: 24-32.
Charoonratana T, Wungsintaweekul J, Keawpradub N, Verpoorte R (2013) Molecular cloning and expression of tryptophan decarboxylase from Mitragyna speciosa. Acta Physiol Plant 35: 2611-2621.
De Luca V., Salim V., Thamm A., Masada S.A., Yu F. (2014) Making iridoids/secoiridoids and monoterpenoid indole alkaloids: Progress on pathway elucidation. Curr Opin Plant Biol. 19: 35-42.
Feng LY, Battulga A, Han E, Chung H, Li JH. (2017) New psychoactive substances of natural origin: A brief review. J. Food Drug Anal. 25: 461-471.
Field E. (1921) XCVIII.- Mitragynine and mitraversine, two new alkaloids from species of Mitragyne. J Chem Soc Trans. 119: 887-891.
Flores-Bocanegra L, Raja HA, Graf TN, Augustinović M, Wallace ED, Hematian S, Kellogg JJ, Todd DA, Cech NB, Oberlies NH (2020) The chemistry of kratom [Mitragyna speciosa]: updated characterization data and methods to elucidate indole and oxindole alkaloids. J Nat Prod. 83: 2165-2177.
Gardner EA, McGrath SA, Dowling D, Bai D (2022) The Opioid Crisis: Prevalence and Markets of Opioids. Forensic Sci Rev. 34: 43-70.
Griffin OH, Webb ME (2018) The scheduling of kratom and selective use of data. J Psychoactive Drugs 50: 114-120.
Gutridge AM, Robins MT, Cassell RJ, Uprety R, Mores KL, Ko MJ, Pasternak GW, Majumdar S, van Rijn RM. (2020) G protein-biased kratom-alkaloids and synthetic carfentanil-amide opioids as potential treatments for alcohol use disorder. Br J Pharmacol. 177: 1497-1513.
Hemby SE, Mclntosh S, Leon F, Cutler SJ, McCurdy CR (2019) Abuse liability and therapeutic potential of the Mitragyna speciosa (kratom) alkaloids mitragynine and 7-hydroxymitragynine. Addict Biol. 24: 874-885.
Jumali SS, Said IM, Baharum SN, Ismail I, Rahman ZA, Zainal Z (2011) Molecular cloning and characterization of strictosidine synthase, a key gene in biosynthesis of mitragynine from Mitragyna speciosa. Afr J Biotech 10: 15238-15244.
Kruegel AC, Gassaway MM, Kapoor A, Varadi A, Majumdar S, Filizola M, et al. (2016) Synthetic and receptor signaling explorations of the Mitragyna alkaloids: mitragynine as an atypical molecular framework for opioid receptor modulators. J Am Chem Soc. 138: 6754-6764.
Kruegel AC, Grundmann O (2018) The medicinal chemistry and neuropharmacology of kratom: A preliminary discussion of a promising medicinal plant and analysis of its potential for abuse. Neuropharmacology 134(A): 108-120.
Leon F, Habib E, Adkins JE, Furr EB, McCurdy CR, Cutler SJ. (2009) Phytochemical characterization of the leaves of Mitragyna speciosa grown in U.S.A. Nat Prod Commun. 4: 907-910.
Mafi A, Kim SK, Goddard WA 3rd. (2020) Mechanism of beta-arrestin recruitment by the mu-opioid G protein-coupled receptor. Proc Natl Acad Sci USA. 117: 16346-16355.
Matsumoto K, Horie S, Ishikawa H, Takayama H, Aimi N, Ponglux D, et al. (2004) Antinociceptive effect of 7-hydroxymitragynine in mice: Discovery of an orally active opioid analgesic from the Thai medicinal herb Mitragyna speciosa. Life Sci. 74: 2143-2155.
Meireles V, Rosado T, Barroso T, Soares S, Gon 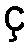 alves J, Luís A, Caramelo D, Simão AY, Fernández N, Duarte AP, Gallardo E (2019) Mitragyna speciosa: Clinical, Toxicological aspects and analysis in biological and non-biological samples. Medicines (Basel) 6: 35.
Palamar JJ (2021) Past-year kratom use in the U.S.: estimates from a nationally representative sample. Am J Prev Med. 61: 240-245.
Prozialeck WC, Jivan JK, Andurkar SV (2012) Pharmacology of kratom: an emerging botanical agent with stimulant, analgesic and opioid-like effects. J Am Osteopath Assoc. 112: 792-799.
Schotte C, Jiang Y, Grzech D, Dang TT, Laforest LC, León F, Mottinelli M, Nadakuduti SS, McCurdy CR, O'Connor SE. (2023) Directed biosynthesis of mitragynine stereoisomers. J Am Chem Soc. 145: 4957-4963.
Sharma A, Kamble SH, León F, Chear NJ, King Tl, Berthold EC, Ramanathan S, McCurdy CR, Avery BA (2019) Simultaneous quantification of ten key Kratom alkaloids in Mitragyna speciosa leaf extracts and commercial products by ultra-performance liquid chromatography-tandem mass spectrometry. Drug Testing & Analysis 11: 1162-1171.
Takayama H (2004) Chemistry and pharmacology of analgesic indole alkaloids from the rubiaceous plant, Mitragyna speciosa. Chem Pharm Bull. 52: 916-928.
Treimer JF, Zenk MH (1979) Purification and properties of strictosidine synthase, the key enzyme in indole alkaloid formation. Eur J Biochem. 101: 225-233.
Váradi A, Marrone GF, Palmer TC, Narayan A, Szabo MR, Le Rouzic V, et al. (2016) Mitragynine/corynantheidine pseudoindoxyls as opioid analgesics with mu agonism and delta antagonism, which do not recruit beta-arrestin-2. J Med Chem. 59: 8381-8397.
Veeramohan R, Azizan KA, Aizat WM, Goh HH, Mansor SM, Yusof NSM, Baharum SN, Nga CL (2018) Metabolomics data of Mitragyna speciosa leaf using LC-ESI-TOF-MS. Data Brief 18: 1212-1216.
Wilson LL, Chakraborty S, Eans SO, Cirino TJ, Stacy HM, Simons CA, et al. (2021) Kratom alkaloids, natural and semi-synthetic, show less physical dependence and ameliorate opioid withdrawal. Cell Mol Neurobiol. 41: 1131-1143.
World Health Organization. Opioid overdose, Key facts. https://www.who.int/news-room/fact-sheets/detail/opioid-overdose. Accessed February 07, 2022
Yusoff NH, Suhaimi FW, Vadivelu RK, Hassan Z, Rumler A, Rotter A, et al. (2016) Abuse potential and adverse cognitive effects of mitragynine (kratom). Addict Biol. 21: 98-110.

## Claims

1. A polypeptide encoding a strictosidine glucosidase that removes the glucose moiety from strictosidine to produce strictosidine aglycone.

2. The polypeptide of claim 1, wherein the strictosidine glucosidase is from *Mitragyna speciosa* and/or wherein the polypeptide comprises or consists of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 1-13, or a fragment of any thereof.

3. A polypeptide encoding a geissoschizine synthase that opens the tetrahydropyran ring of strictosidine aglycone to produce geissoschizine.

4. The polypeptide from claim 3, wherein the geissoschizine synthase is from *Mitragyna speciosa* and/or wherein the polypeptide comprises or consists of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 14-22, or a fragment of any thereof.

5. A polypeptide encoding a hydroxylase that hydroxylates the indole ring of coηnantheidine at the C9 position to produce 9-hydroxycorynantheidine.

6. A polypeptide encoding a hydroxylase that hydroxylates the indole ring of mitragynine at the C7 position to produce 7-hydroxymitragynine.

7. The polypeptide of claims 5 and 6, wherein the hydroxylase is from *Mitragyna speciosa* and/or wherein the polypeptide comprises or consists of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 23-54, or a fragment of any thereof.

8. A polypeptide encoding an O-methyltransferase that methylates the hydroxyl group at the C9 position of 9-hydroxycorynantheidine to produce mitragynine.

9. The polypeptide of claim 8, wherein the O-methyltransferase is from *Mitragyna speciosa* and/or wherein the polypeptide comprises or consists of a polypeptide variant having at least 80%, 85%, 90%, 91%, 92%, 93%, 94%, 95%, 96%, 97%, 98%, 99%, or 100% sequence identity to the sequence of any one or more of SEQ ID NO: 55-65, or a fragment of any thereof.

10. A system for modifying an indole alkaloid, the system comprising the polypeptide of any one of claims 1 to 9.

11. The system of claim 10, wherein the system is cell-free.

12. A method for modifying an indole alkaloid, wherein the method comprises contacting the indole alkaloid with the polypeptide of any one of claims 1 to 9.

13. The method of claim 12, wherein the method is a recombinant method comprising expressing a polypeptide from any one of claims 1 to 9 in a cell in the presence of an indole alkaloid precursor.

14. A method of producing an indole alkaloid selected from strictosidine aglycone, 4,21-dehydrogeissoschizine, geissoschizine, corynantheidine, 9-hydroxycorynantheidine, mitragynine, 7-hydroxymitragynine, speciociliatine, speciogynine, and paynantheine, the method comprising carrying out combined enzymatic reactions with the polypeptides from any one of claims 1 to 9.

15. The indole alkaloid of claim 14, wherein the indole alkaloid is substantially pure, for example, at least about 95%, about 96%, about 97%, about 98%, about 99%, about 99.5%, or about 99.9% pure.
